# EUROPÄISCHE PATENTANMELDUNG

(11) **EP 2 098 262 A1**
(43) Veröffentlichungstag der Anmeldung: **09.09.2009**
(21) Anmeldenummer: 09152391.0
(22) Anmeldetag: 09.02.2009
(51) Int. Cl.: A61M 25/09

(54) **Katheter-Führungsdraht und Verfahren zur Herstellung desselben**

(30) Priorität: 05.03.2008 DE 102008012743
(71) Anmelder: Biotronik VI Patent AG, 6341 Baar (CH)
(72) Erfinder: Hofmann, Eugen, 8049, Zürich (CH)
(74) Vertreter: Lindner-Vogt, Karin L.

(57) **Zusammenfassung**

Die Erfindung betrifft einen Katheter-Führungsdraht (1), vorzugsweise für kardio-vaskuläre Eingriffe, mit einem distalen Ende (2) und einem proximalen Ende (3) und einen sich im Wesentlichen vom distalen zum proximalen Ende erstreckenden, einstückigen Kern (10). Die Erfindung zeichnet sich dadurch aus, dass der Kern verdrillte Fasern, vorzugsweise Glasfasern, aufweist, die in das Grundmaterial des Kerns eingebettet sind. Die Erfindung betrifft außerdem ein Verfahren zur Herstellung eines derartigen Katheter-Führungsdrahts.

## Beschreibung

Die vorliegende Erfindung betrifft einen Katheter-Führungsdraht, vorzugsweise für kardio-vaskuläre Eingriffe mit einem distalen und einem proximalen Ende und einen sich im Wesentlichen vom distalen zum proximalen Ende erstreckenden, einstückigen Kern. Die Erfindung betrifft ferner ein Verfahren zur Herstellung eines derartigen Katheter-Führungsdrahts.

Beim Einsatz von Kathetern, z. B. im Rahmen von kardio-vaskulären Eingriffen, wird der Katheter mit Hilfe eines vorher in den Körper des Patienten eingeführten Führungsdrahts positioniert. Die distale Spitze eines Katheter-Führungsdrahts ist in der Regel sehr flexibel ausgebildet, so dass diese gebogen und gedreht werden kann, um zu der gewünschten Stelle im Körper des Patienten, z. B. innerhalb eines Blutgefäßes, vorgeschoben werden zu können.

In der Druckschrift WO 98/42268 A1 wird ein Führungsdraht offenbart, der einen Kern aus einem Glaskörper aufweist. Der Glaskern besteht beispielsweise aus Fiberglas, Silizium oder Quarz und ist von einer Beschichtung umgeben. Der Glaskern mit Beschichtung, welche zur Verstärkung des Glaskerns dient, erstreckt sich im Wesentlichen über die Länge des Führungsdrahts und ist von einer Kunststoffumhüllung umgeben, welche an dem Glaskörper mit Beschichtung mit einem Kleber befestigt ist. Die äußere Kunststoffumhüllung ist dafür vorgesehen, die physikalische Integrität des Führungsdrahts aufrechtzuerhalten, selbst wenn der Glaskern brechen sollte. Hierfür weist die Umhüllung beispielsweise Verstärkungsfasern auf, wobei die Umhüllung mit den Verstärkungsfasern geflochten sein kann. Die Verstärkungsfasern können auch in einer Polymermatrix angeordnet sein. Als Material für die Verstärkungsfasern dienen beispielsweise Kohlenstoff, Aramide oder Glas.

In der Druckschrift DE 10 2004 028 367 A1 wird ein Katheter-Führungsdraht offenbart, dessen langgestreckter Kern einstückig ausgebildet ist und vom proximalen bis zum distalen Ende durchgehend aus dem Kunststoff PEEK besteht. Der Kern ist im Endbereich seines distalen Endes im Durchmesser gegenüber dem Durchmesser am proximalen Ende verjüngt ausgebildet. Der Führungsdraht weist eine Beschichtung auf, in der ein MRT-Kontrastmittel vorgesehen ist. Als MRT-Kontrastmittel können Elemente der Lanthaniden-Gruppe, wie Gadolinium oder Dysprosium, verwendet werden. Die MRT-Kontrastmittel dienen zur Überwachung des Vorschubes des Führungsdrahts z. B. durch die Gefäße bis zu dem Zielort der Behandlung. Hierdurch wird die Überwachung mittels eines Magnetresonanztomographie-Geräts (im Folgenden auch MRT-Scanner genannt) ermöglicht, welches für den Patienten belastungsfrei Bildinformationen liefert.

Der bekannte Führungsdraht mit PEEK-Kern hat jedoch den Nachteil, dass er für einige Anwendungen nicht steif genug ist. Insbesondere kann ein derartiger PEEK-Führungsdraht aufgrund des sogenannten "Kriechens" des Kunststoffs nach der Lagerung in einer Spirale bleibend deformiert vorliegen, so dass die für den Einsatz des Führungsdrahts erforderliche Geradheit des Führungsdrahts nicht gegeben ist. Der Führungsdraht ist in diesem Fall in der klinischen Anwendung nur noch bedingt einsetzbar. Zudem ergeben die Partikel in der Polymerbeschichtung nur eine diffuse und somit eine ungenügende Visualisierung im MRT-Scanner.

Die Aufgabe der vorliegenden Erfindung besteht somit darin, einen Katheter-Führungsdraht zu schaffen, dessen Geradheit auch nach einer Lagerzeit in einer runden Verpackung, wo der Führungsdraht z. B. in einer kreisrunden Anordnung vorliegt, erhalten bleibt. In Bezug auf die Flexibilität bzw. Steifheit soll der Führungsdraht ebenfalls sehr gute Eigenschaften aufweisen. Ferner muss die erforderliche Knickresistenz gewährleistet sein. Die Aufgabe besteht ferner darin, ein einfaches und kostengünstiges Verfahren zur Herstellung eines derartigen Führungsdrahts anzugeben.

Die oben angegebene Aufgabe wird durch einen Katheter-Führungsdraht gelöst, dessen Kern verdrillte Fasern, vorzugsweise Glasfasern, aufweist, die in das Grundmaterial des Kerns eingebettet sind.

Durch die Verwendung verdrillter Fasern, wobei jede der verdrillten Fasern sich vorzugsweise im Wesentlichen über die gesamte Länge des Kerns erstreckt, wobei die Fasern bevorzugt unter axialem Zug und Wärmeeinwirkung verdrillt sind, wird gewährleistet, dass der Führungsdraht die nötige Steifheit aufweist, da die verdrillten Fasern unter einer axialen Spannung verdrillt wurden. Aufgrund der erhöhten Steifheit bleibt der Führungsdraht auch nach längerer Lagerung in einer z. B. runden Verpackung gerade.

In einem bevorzugten Ausführungsbeispiel beträgt der Faser-Anteil am Gesamtmaterial des Kerns, das aus dem Grundmaterial und den Fasern besteht, mindestens etwa 30 Volumen%, bevorzugt mindestens 40 Volumen%. Hierdurch weist der Führungsdraht eine derart gute Flexibilität bzw. Steifheit auf, so dass die Bewegung des Führungsdrahts, beispielsweise in Gefäßen, nicht behindert wird.

Das Material der Fasern umfasst bevorzugt SiO₂, wobei in einem besonders bevorzugten Ausführungsbeispiel das Fasermaterial mindestens überwiegend aus Saint-Gobain Quartzel-Fasern mit einem Durchmesser von mindestens 10 µm gebildet ist. Durch eine derartige Wahl des Fasermaterials wird die notwendige Knickresistenz des Katheter-Führungsdrahts erreicht.

Bevorzugt weist der Kern eine erste Beschichtung auf, die den Kern umhüllt, wobei die erste Beschichtung vorzugsweise mindestens ein Material der Gruppe bestehend aus Polyurethan und Polyamid umfasst.

Bevorzugt ist der erfindungsgemäße Führungsdraht so ausgebildet, dass der Kern in einem Endabschnitt an seinem distalen Ende einen geringeren Durchmesser und die erste Beschichtung an diesem Ende eine entsprechend erhöhte Dicke verglichen mit dem Durchmesser des Kerns am proximalen Ende bzw. der Dicke der ersten Beschichtung am proximalen Ende des Führungsdrahts aufweist. Der geringere Durchmesser des Kerns und die erhöhte Dicke der Beschichtung im Bereich des distalen Endes führt dazu, dass der Führungsdraht am distalen Ende eine größere Flexibilität hat. Besonders bevorzugt hierbei ist, dass das Verhältnis von proximalem Durchmesser (Durchmesser im Schaftabschnitt) zu distalem Durchmesser (Durchmesser im distalen Abschnitt) des Kerns etwa 2:1 bis 5:1, vorzugsweise etwa 3:1, beträgt.

Um einen optimalen Übergang von dem weichen distalen Ende des Führungsdrahts zum steifen proximalen Ende zu erzielen, weist der Kern in einem bevorzugten Ausführungsbeispiel ein konischer Abschnitt auf, wobei der konische Abschnitt zwischen dem distalen Abschnitt des Kerns, der am weitesten distal angeordnet ist, und dem Schaftabschnitt des Kerns liegt, der am weitesten proximal angeordnet ist. Die Länge dieses konischen Abschnitts in Längsrichtung beträgt mindestens etwa 50 mm, vorzugweise mindestens etwa 100 mm. Der Endabschnitt des Kerns an seinem distalen Ende setzt sich somit aus dem distalen Abschnitt und dem konischen Abschnitt zusammen.

Um eine passive Visualisierung in einem MRT-Scanner zu ermöglichen, weist der Kern mindestens eine Markierung aus einem MR-sichtbaren Material auf. Eine derartige Markierung wird im Folgenden auch als MR-Marker bezeichnet.

Vorzugsweise weist der MR-Marker mindestens ein Material der Gruppe bestehend aus Eisen, Titan, den Elementen der Gruppe der Lanthaniden, vorzugsweise Gadolinium oder Dysprosium, auf. In einem weiteren bevorzugten Ausführungsbeispiel weist der Kern zwischen dem distalen und dem proximalen Ende mehrere, vorzugsweise kugel-, zylinder- oder hohlzylinderförmige, Markierungen auf, die vorzugsweise in definierten Abständen angeordnet sind. Besonders bevorzugt ist, wenn das Gesamtvolumen der Markierungen in Richtung des distalen Endes des Kerns zunimmt.

In einem weiteren Ausführungsbeispiel ist mindestens am distalen Ende des Kerns, bevorzugt im Bereich des distalen Abschnitts, zwischen Kern und erster Beschichtung ein plättchenförmiges Steuerelement vorgesehen, das vorzugsweise aus rostfreiem Stahl besteht. Mittels eines derartigen, langgestreckten Flachdrahts, der auch als MR-Marker dienen kann und in der distalen Spitze des Führungsdrahts angeordnet ist, kann der Führungsdraht vorgebogen und gleichzeitig im MR-Scanner sichtbar gemacht werden.

In einem weiteren Ausführungsbeispiel ist auf der ersten Beschichtung eine zweite, hydrophile Beschichtung angeordnet, welche sich vorzugsweise nicht bis zum proximalen Ende des Führungsdrahts erstreckt. Die hydrophile Beschichtung bewirkt, dass der Führungsdraht gute Gleiteigenschaften aufweist, während er am proximalen Ende, das keine hydrophile Beschichtung aufweist, griffig bleibt.

Die obige Aufgabenstellung wird ferner gelöst durch ein Verfahren zur Herstellung eines Katheter-Führungsdrahts mit den folgenden Schritten:
a) Pultrudieren der Fasern mit dem Grundmaterial des Kerns,
b) Verdrillen des in Schritt a) erhaltenen faserverstärkten Materials unter axialem Zug und einer Wärmebehandlung,
c) Schleifen des verdrillten faserverstärkten Materials auf den oder die gewünschten Durchmesser des Kerns und
d) Aufbringen der ersten Beschichtung auf das faserverstärkte Material des Kerns.

Das erfindungsgemäße Herstellungsverfahren ist einfach und kostengünstig und ergibt einen Führungsdraht, welcher eine erhöhte Steifheit besitzt, so dass dessen Geradheit auch bei längerer Lagerung nicht verloren geht. Vorzugsweise ist der Schleifprozess des erfindungsgemäßen Verfahrens in Schritt c) so dimensioniert, dass am distalen Ende der Durchmesser des Kerns mittels Schleifen so weit reduziert wird, dass eine weiche und atraumatische Spitze des Führungsdrahts realisiert wird.

Es ist außerdem bevorzugt, dass die erste Beschichtung auf den Kern aufextrudiert wird. Die erste Beschichtung, die vorzugsweise aus einem Kunststoff besteht, schützt den Führungsdraht, da er bei Knicken oder Brechen des Kerns immer noch durch den Überzug bestehend aus der ersten Beschichtung zusammengehalten wird und keine Teile des Kerns in dem Gefäß, in das der Führungsdraht eingeführt ist, weggeschwemmt werden können.

In einem weiteren Ausführungsbeispiel kann auf die erste Beschichtung außerdem eine zweite, hydrophile Beschichtung aufgebracht werden, welche sich nicht bis zum proximalen Ende des Führungsdrahts erstreckt, sondern im Wesentlichen am distalen Ende des Kerns angeordnet ist und die erste Beschichtung umhüllt.

Besonders bevorzugt ist, wenn beim Verdrillen in Schritt b) maximal 1/4 Umdrehungen pro 10 mm Länge in Längsrichtung der Fasern erzeugt werden, um die Knickresistenz des Katheter-Führungsdrahts nicht herabzusetzen.

Weitere Ziele, Merkmale, Vorteile und Anwendungsmöglichkeiten der Erfindung ergeben sich aus der nachfolgenden Beschreibung eines Ausführungsbeispiels anhand der Figuren. Dabei bilden alle beschriebenen und/oder bildlich dargestellten Merkmale für sich oder in beliebiger Kombination den Gegenstand der vorliegenden Erfindung, auch unabhängig von ihrer Zusammenfassung in einzelnen Ansprüchen oder deren Rückbeziehung.

Es zeigt schematisch:
- Figur 1: einen Längsschnitt eines ersten Ausführungsbeispiels eines erfindungsgemäßen Führungsdrahts und
- Figur 2: einen Längsschnitt eines zweiten Ausführungsbeispiels eines erfindungsgemäßen Führungsdrahts.

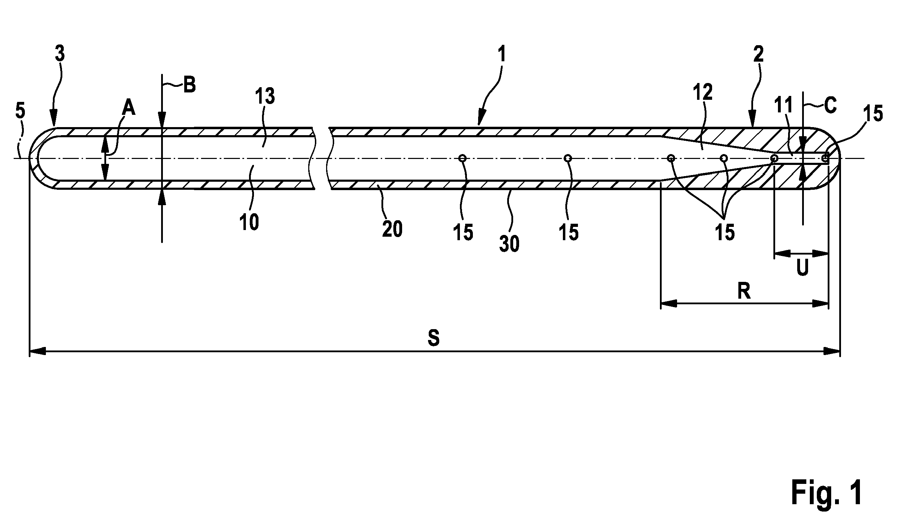

In Figur 1 wird ein erfindungsgemäßer Katheter-Führungsdraht 1 mit einem distalen Ende 2 und einem proximalen Ende 3 gezeigt. Der Führungsdraht 1 weist einen im Wesentlichen zylinderförmigen Kern 10 auf, dessen Grundmaterial aus dem Kunststoff PEEK (Polyether-Etherketon) besteht, das mit verdrilltem Saint-Gobain Quartzel-Fasern mit einem Durchmesser von etwa 10 µm versehen ist. Der Kern 10 erstreckt sich in Längsrichtung entlang der Achse 5. Der Kern 10 ist von einer Kunststoff-Beschichtung (erste Beschichtung) 20 ummantelt, welche aus Polyurethan (z. B. PU Pellethane Shore 90 A) besteht.

Die Beschichtung 20 ist wiederum von einer zweiten, hydrophilen Beschichtung 30 umgeben, welche sich über die Länge des Kerns, insbesondere seinem distalen Endabschnitt, bis auf beispielsweise die proximalsten 20 cm der Länge des Führungsdrahts 1 erstreckt. Vor dem Einsatz weist die hydrophile Beschichtung etwa eine Dicke von 10 µm auf. Beim Einsatz quillt die Beschichtung sehr stark. Die hydrophile Beschichtung besteht beispielsweise aus einer Lösung aus photo-reactivem Polyvinylpyrrolidone-Copolymer.

Der Kern 10 ist zusammengesetzt aus drei Abschnitten: dem distalen Abschnitt 11, dem konischen Abschnitt 12 und dem Schaftabschnitt 13, die in dieser Reihenfolge hintereinander vom distalen Ende des Kerns 10 aus gesehen angeordnet sind. Im distalen Abschnitt 11 ist der Durchmesser C des Kerns 10 am geringsten und im Schaftabschnitt 13 am größten. Im konischen Abschnitt 12 geht der Durchmesser des Kerns 10 von dem geringeren Wert im distalen Abschnitt 11 zu dem größeren Wert im Schaftabschnitt 13 über.

In einem bevorzugten Ausführungsbeispiel weist der Führungsdraht eine Länge S von etwa 160 cm auf. Die Länge R des distalen Endabschnitts des Kerns 10, bestehend aus dem distalen Abschnitt 11 und dem konischen Abschnitt 12 beträgt etwa 120 mm. Die Länge des distalen Abschnitts 11 (auch Floppy Tip genannt) beträgt etwa 20 mm und die Länge des konischen Abschnitts 12 etwa 100 mm. Der Durchmesser C des Kerns im distalen Abschnitt beträgt etwa zwischen 150 und 250 µm, wobei der Durchmesser C über den gesamten distalen Abschnitt 11 konstant ist. Der Durchmesser A des Kerns im Bereich des proximalen Endes des Führungsdrahts 1 (Schaftabschnitt 13) beträgt etwa 0,57 mm. Der äußere Durchmesser des Führungsdrahts B in dem Bereich des Führungsdrahts an seinem proximalen Ende, in dem keine zweite Beschichtung 30, sondern lediglich Kern 10 und erste Beschichtung 20 vorgesehen sind, beträgt etwa 0,88 mm.

Auf dem Kern 10 sind MR-Marker 15 aus runder rostfreier Stahlfolie zur passiven Visualisierung im MR-Scanner eingebracht, welche zunächst in einem geringeren Abstand und, weiter entfernt von dem distalen Ende 2 des Führungsdrahts, in einem weiteren Abstand angeordnet sind. Beispielsweise sind sechs MR-Marker 15 vorgesehen, die ausgehend vom distalen Ende des Kerns im Abstand von 2 cm und zwei MR-Marker 15, die im Abstand von 4 cm angeordnet sind. Die MR-Marker 15 wurden aus runden Metallfolien mit einem Durchmesser von 1,1 mm gestanzt und um den Kern 10 montiert.

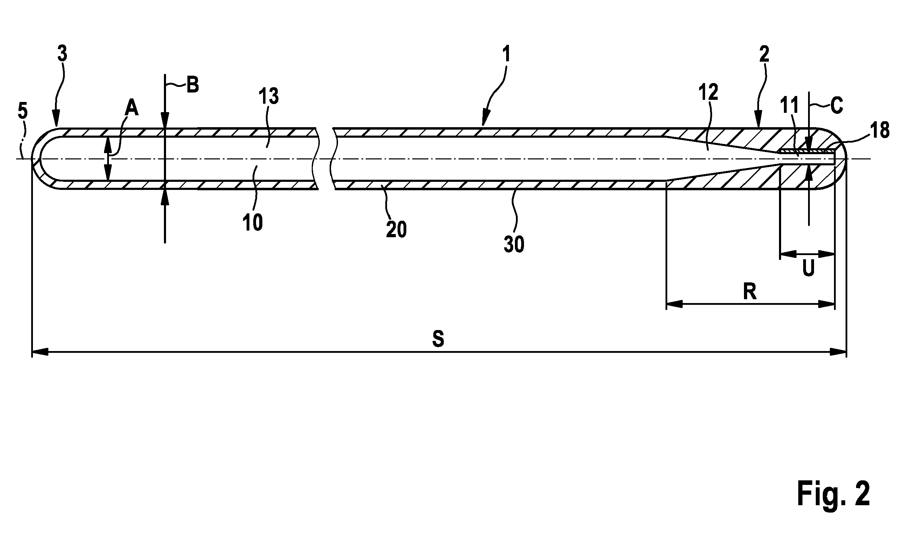

In einem weiteren Ausführungsbeispiel, das in Figur 2 gezeigt ist, weist der Führungsdraht anstelle der Markierungen 15 im distalen Abschnitt 11 des Kerns 10 zwischen dem Kern 10 und der ersten Beschichtung 20 ein plättchenförmiges Steuerelement 18 in Form eines Flachdrahts aus rostfreiem Stahl auf. Dieses Steuerelement 18 dient einerseits zur Bewegung der distalen Spitze 2 des Führungsdrahts 1 und andererseits zur Erkennung der Position des Führungsdrahts 1 im MR-Scanner. Die Länge des Steuerelements 18 in Längsrichtung des Katheter-Führungsdrahts 1 beträgt etwa 20 bis 50 mm.

### Bezugszeichenliste

- 1: Katheter-Führungsdraht
- 2: distales Ende des Führungsdrahts 1
- 3: proximales Ende des Führungsdrahts 1
- 5: Längsachse des Führungsdrahts 1
- 10: Kern
- 11: distaler Abschnitt des Kerns 10
- 12: konischer Abschnitt des Kerns 10
- 13: Schaftabschnitt des Kerns 10
- 15: MR-Marker, Markierung
- 18: Flachdraht, Steuerelement
- 20: erste Beschichtung
- 30: zweite, hydrophile Beschichtung
- A: Durchmesser des Kerns 10 am proximalen Ende, d. h. im Schaftabschnitt 13
- B: Gesamtdurchmesser des Führungsdrahts 1 am proximalen Ende 3
- C: Durchmesser des Kerns 10 im Bereich des distalen Abschnitts 11
- S: Gesamtlänge des Führungsdrahts 1
- R: Länge des distalen Endabschnitts des Kerns 10, bestehend aus distalem Abschnitt 11 und konischem Abschnitt 12
- U: Länge des distalen Abschnitts 11 des Kerns 10, in dem der Kern 10 einen konstanten Durchmesser aufweist

## Patentansprüche

1. Katheter-Führungsdraht (1), vorzugsweise für kardio-vaskuläre Eingriffe, mit einem distalen Ende (2) und einem proximalen Ende (3) und einem sich im Wesentlichen vom distalen zum proximalen Ende erstreckenden, einstückigen Kern (10), **dadurch gekennzeichnet, dass** der Kern verdrillte Fasern, vorzugsweise Glasfasern, aufweist, die in das Grundmaterial des Kerns eingebettet sind.

2. Katheter-Führungsdraht nach Anspruch 1, **dadurch gekennzeichnet, dass** sich jede der verdrillten Fasern im Wesentlichen über die gesamte Länge des Kerns (10) erstreckt, wobei die Fasern bevorzugt unter axialem Zug und Wärmeeinwirkung verdrillt sind.

3. Katheter-Führungsdraht nach einem der Ansprüche 1 oder 2, **dadurch gekennzeichnet, dass** der Faser-Anteil am Gesamtmaterial des Kerns (10) mindestens etwa 30 Volumen%, bevorzugt mindestens etwa 40 Volumen% beträgt.

4. Katheter-Führungsdraht nach einem der vorhergehenden Ansprüche, **dadurch gekennzeichnet, dass** das Material der Fasern SiO₂ umfasst, wobei das Fasermaterial bevorzugt mindestens überwiegend aus Saint-Gobain Quartzel-Fasern mit einem Durchmesser von mindestens 10 µm gebildet ist.

5. Katheter-Führungsdraht nach einem der vorhergehenden Ansprüche, **dadurch gekennzeichnet, dass** das Grundmaterial des Kerns (10) mindestens einen Kunststoff umfasst, wobei als Kunststoffmaterial bevorzugt PEEK verwendet wird.

6. Katheter-Führungsdraht nach einem der vorhergehenden Ansprüche, **dadurch gekennzeichnet, dass** der Kern (10) eine erste Beschichtung (20) aufweist, wobei die erste Beschichtung vorzugsweise mindestens ein Material der Gruppe bestehend aus Polyurethan und Polyamid umfasst.

7. Katheter-Führungsdraht nach Anspruch 6, **dadurch gekennzeichnet, dass** der Kern (10) in einem Endabschnitt (11, 12) an seinem distalen Ende einen geringeren Durchmesser (C) und die erste Beschichtung (20) eine entsprechend erhöhte Dicke jeweils verglichen mit dem Durchmesser (A) des Kerns bzw. der Dicke der ersten Beschichtung am proximalen Ende (3) des Führungsdrahts aufweisen.

8. Katheter-Führungsdraht nach Anspruch 7, **dadurch gekennzeichnet, dass** der Endabschnitt des Kerns an seinem distalen Ende einen distalen Abschnitt (11) und einen konischen Abschnitt (12) aufweist, wobei der konische Abschnitt zwischen dem distalen Abschnitt und einem Schaftabschnitt (13) des Kerns angeordnet ist und der konische Abschnitt eine Länge in Längsrichtung von mindestens etwa 50 mm, vorzugsweise mindestens etwa 100 mm aufweist.

9. Katheter-Führungsdraht nach einem der vorhergehenden Ansprüche, **dadurch gekennzeichnet, dass** der Kern (10) mindestens eine Markierung (15) aus einem MR-sichtbaren Material aufweist.

10. Katheter-Führungsdraht nach Anspruch 9, **dadurch gekennzeichnet, dass** die Markierung (15) mindestens ein Material der Gruppe bestehend aus Eisen, Titan, Elemente der Gruppe der Lanthaniden, vorzugsweise Gadolinium oder Dysprosium, aufweist.

11. Katheter-Führungsdraht nach einem der Ansprüche 9 oder 10, **dadurch gekennzeichnet, dass** der Kern (10) zwischen dem distalen und dem proximalen Ende mehrere, vorzugsweise kugel-, zylinder- oder hohlzylinderförmige Markierungen (15) aufweist, wobei das Gesamtvolumen der Markierungen in Richtung des distalen Endes des Kerns zunimmt.

12. Katheter-Führungsdraht nach einem der vorhergehenden Ansprüche, **dadurch gekennzeichnet, dass** zwischen Kern (10) und erster Beschichtung (20) im Bereich des distalen Abschnitts (11) ein blättchenförmiges Steuerelement (18) vorgesehen ist, das bevorzugt aus rostfreiem Stahl besteht und/oder dass mindestens am distalen Ende des Kerns (10) auf der ersten Beschichtung (20) eine zweite, hydrophile Beschichtung (30) angeordnet ist.

13. Verfahren zur Herstellung eines Katheter-Führungsdraht nach einem der vorhergehenden Ansprüche mit den folgenden Schritten:
a) Pultrudieren der Fasern mit dem Grundmaterial des Kerns (10),
b) Verdrillen des in Schritt a) erhaltenen faserverstärkten Materials unter axialem Zug und einer Wärmebehandlung,
c) Schleifen des verdrillten faserverstärkten Materials auf den oder die gewünschten Durchmesser des Kerns (10) und
d) Aufbringen der ersten Beschichtung (20) auf das faserverstärkte Material des Kerns (10).

14. Verfahren nach Anspruch 13, **dadurch gekennzeichnet, dass** die erste Beschichtung (20) auf den Kern (10) aufextrudiert wird und/oder dass auf die erste Beschichtung (20) am distalen Ende des Kerns (10) eine zweite, hydrophile Beschichtung (30) aufgebracht wird.

15. Verfahren nach Anspruche 13 oder 14, **dadurch gekennzeichnet, dass** das Verdrillen im Schritt b) mit maximal 1/4 Umdrehung pro 10 mm Länge in Längsrichtung erfolgt.
